# EUROPEAN PATENT APPLICATION

(11) **EP 4 407 039 A1**
(43) Date of publication of application: **31.07.2024**
(21) Application number: 22872950.5
(22) Date of filing: 21.09.2022
(51) Int. Cl.: C12P 19/14, C12N 1/15, C12N 15/31, C12N 15/56, C12N 9/30, C12N 9/42

(54) **METHOD FOR PRODUCING SACCHARIFYING ENZYME**

(30) Priority: 22.09.2021 JP 2021154749
(71) Applicant: Kao Corporation, Tokyo 103-8210 (JP)
(72) Inventor: MAEDA, Hiromi, Wakayama-shi, Wakayama 640-8580 (JP); KODAMA, Takeko, Tokyo 131-8501 (JP); SHIBATA, Nozomu, Wakayama-shi, Wakayama 640-8580 (JP); TAKAHASHI, Fumikazu, Wakayama-shi, Wakayama 640-8580 (JP); IGARASHI, Kazuaki, Tokyo 131-8501 (JP)
(74) Representative: Vossius & Partner Patentanwälte Rechtsanwälte mbB
(86) International application number: PCT/JP2022/035249
(87) International publication number: WO 2023/048199

(57) **Abstract**

Provided is a method for producing a saccharifying enzyme which prevents an increase in viscosity caused by pectin in the biomass saccharification process.

A method for producing a biomass saccharifying enzyme, comprising culturing a recombinant filamentous fungus into which one or both of a polygalacturonase gene selected from the following gene group (a) and a pectin lyase gene selected from the following gene group (b) are introduced:
(a) PgaB gene, PgaI gene, and PgaII gene; and
(b) PelD gene and PelF gene.

## Description

### Field of the Invention

The present invention relates to a method for producing a biomass saccharifying enzyme.

### Background of the Invention

Due to the depletion of fossil resources used for energy and materials, and environmental problems such as global warming, technological development to replace fossil resources with renewable resources is being carried out all over the world. In particular, advanced use of cellulose-based biomass is required from the viewpoint of carbon neutrality, and the use of industrial waste is expected.

Cassava is produced in Africa and Asia, and its starch is extracted for use as tapioca starch, mainly in Asia. The cassava residue after extracting tapioca starch has a water content of from 70 to 90% and is used as livestock feed after drying; however, not the whole residue can be used, and the rest is discarded. However, since cassava residue contains a large amount of starch and cellulose and is a biomass with an abundant amount of glucose, it is expected to be used in advanced ways by converting it into sugar. On the other hand, cassava residue is known to contain a large amount of pectin in addition to starch and cellulose. Because pectin is a natural polysaccharide which is a polymer mainly present in cell walls, it has the property of high viscosity.

In order to convert cellulose and the like in cassava residue into sugar, a saccharification process using a biomass saccharifying enzyme composed of cellulase and the like is required; however, the issue is that increased viscosity due to pectin affects the conversion efficiency into sugar. In addition, cassava residue contains hemicellulose and lignin in addition to starch, cellulose, and pectin, and multiple enzymes such as amylase, cellulase, pectinase, and hemicellulase are essential for saccharification.

Non-Patent Literature 1 discloses that an enzyme mixture containing cellulase, hemicellulase, and pectinase derived from *Aspergillus aculeatus* can reduce the viscosity of cassava roots, chips, and pulp. In addition, Patent Literature 1 discloses that an enzyme preparation containing cellulase and pectinase can reduce the water content and material viscosity in cassava residue and increase the yield of starch.

However, saccharifying biomass using a mixture of various enzymes is extremely expensive, making these techniques impractical for carrying out saccharification of cassava residue on a large scale. Techniques have been required to produce saccharifying enzymes inexpensively.

[Patent Literature 1] CN-A-101285058
[Non-Patent Literature 1] Aphisit Poonsrisawat et al., Process Biochemistry 49 (2014) 1950-1957

### Summary of the Invention

The present invention relates to the following 1) and 2).
1) A method for producing a biomass saccharifying enzyme, comprising culturing a recombinant filamentous fungus into which one or both of a polygalacturonase gene selected from the following gene group (a) and a pectin lyase gene selected from the following gene group (b) are introduced:
   (a) PgaB gene, PgaI gene, and PgaII gene; and
   (b) PelD gene and PelF gene.
2) A method for biomass saccharification, comprising using, as a biomass saccharifying agent, a culture obtained by culturing a recombinant filamentous fungus into which one or both of a polygalacturonase gene selected from the following gene group (a) and a pectin lyase gene selected from the following gene group (b) are introduced:
   (a) PgaB gene, PgaI gene, and PgaII gene; and
   (b) PelD gene and PelF gene.

### Detailed Description of the Invention

The present invention relates to provision of a method for producing a saccharifying enzyme which prevents an increase in viscosity caused by pectin in the biomass saccharification process.

The present inventors found that a saccharifying enzyme which exhibits an excellent preventive effect on an increase in the viscosity of biomass caused by pectin can be efficiently produced by culturing a recombinant filamentous fungus into which a specific pectin-degrading enzyme (pectinase) gene is introduced.

The present invention makes it possible to inexpensively produce a saccharifying enzyme which prevents an increase in the viscosity of slurry in the saccharification process of biomass containing pectic substances, and which allows for efficient saccharification process.

### (1. Definition)

In the present invention, the identity of amino acid sequences or nucleotide sequences is calculated by the Lipman-Pearson method (Science, 1985, 227: 1435-1441). Specifically, the identity is calculated by performing analysis using the homology analysis (Search homology) program of genetic information processing software GENETYCS Ver. 12 while setting the unit size to compare (ktup) to 2.

In the present invention, the phrase "at least 90% identity" relating to amino acid sequences or nucleotide sequences refers to an identity of 90% or more, preferably 95% or more, more preferably 96% or more, even more preferably 97% or more, still even more preferably 98% or more, and further still more preferably 99% or more.

Examples of an amino acid sequence or nucleotide sequence having at least 90% identity to a certain amino acid sequence or nucleotide sequence include an amino acid sequence having deletion, substitution, addition, or insertion of one or more amino acids, or a nucleotide sequence having deletion, substitution, addition, or insertion of one or more nucleotides.

The "amino acid sequence having deletion, substitution, addition, or insertion of one or more amino acids" as mentioned herein refers to an amino acid sequence having deletion, substitution, addition, or insertion of 1 or more and 10 or less, preferably 1 or more and 8 or less, more preferably 1 or more and 5 or less, and even more preferably 1 or more and 3 or less amino acids. Further, the "nucleotide sequence having deletion, substitution, addition, or insertion of one or more nucleotides" refers to a nucleotide sequence having deletion, substitution, addition, or insertion of 1 or more and 30 or less, preferably 1 or more and 24 or less, more preferably 1 or more and 15 or less, and even more preferably 1 or more and 9 or less nucleotides. In the present specification, the "addition" of amino acids or nucleotides includes addition of amino acids or nucleotides to one end and both ends of the sequence.

In the present invention, the terms "upstream" and "downstream" relating to a gene refer respectively to the upstream and downstream of the gene in the transcription direction. For example, "a gene located downstream of the promoter" means that the gene is present on the 3' side of the promoter in the DNA sense strand, and the upstream of a gene refers to the region on the 5' side of the gene in the DNA sense strand.

In the present invention, the "operable linkage" between a control region and a gene means that the gene and the control region are linked such that the gene can be expressed under the control of the control region. The procedure of the "operable linkage" between the gene and the control region is well known to a person skilled in the art.

In the present invention, the phrase "a gene corresponding to" refers to, for a gene encoding an enzyme which catalyzes a given reaction in a microorganism, a gene encoding an enzyme which catalyzes the same or similar reaction as that enzyme in another microorganism. A gene and its corresponding gene may have the same nucleotide sequence or different nucleotide sequences; however, the nucleotide sequence identity between a gene and its corresponding gene is preferably at least 90%.

### (2. Recombinant Filamentous Fungus and Production Thereof)

The recombinant filamentous fungus of the present invention is a filamentous fungus into which a specific pectinase gene is introduced. Specifically, the recombinant filamentous fungus of the present invention is a filamentous fungus into which one or both of a polygalacturonase gene selected from the following gene group (a) and a pectin lyase gene selected from the following gene group (b) are introduced:
(a) PgaB gene, PgaI gene, and PgaII gene; and
(b) PelD gene and PelF gene.

"Polygalacturonase" is an enzyme which hydrolyzes the α-1,4-glycosidic bond of D-galacturonic acid, which constitutes pectin acid (EC3.2.1.15), and examples of the polygalacturonase gene of the present invention include one or more genes selected from the group consisting of PgaB gene, PgaI gene, and PgaII gene.

Such a polygalacturonase gene is preferably derived from a filamentous fungus, and preferred examples thereof include those derived from microorganisms of the genus *Aspergillus* (e.g., *Aspergillus niger, Aspergillus japonicus,* and *Aspergillus aculeatus*), microorganisms of the genus *Rhizopus* (e.g., *Rhizopus oryzae* and *Rhizopus delemar*), microorganisms of the genus *Talaromyces* (e.g., *Talaromyces cellulolyticus*), microorganisms of the genus *Penicillium* (e.g., *Penicillium roqueforti*), and the like.

More preferred among these are PgaB gene of *Aspergillus niger* or a gene corresponding to the PgaB gene, PgaI gene of *Aspergillus niger* or a gene corresponding to the PgaI gene, PgaII gene of *Aspergillus niger* or a gene corresponding to the PgaII gene, and the like.

The PgaB gene of *Aspergillus niger* is registered as accession number XM_025597756 in the NCBI public database. Specific examples of the PgaB gene of *Aspergillus niger* or the gene corresponding to the PgaB gene include the following 1) to 3):
1) a polynucleotide consisting of a nucleotide sequence of SEQ ID NO: 1;
2) a polynucleotide consisting of a nucleotide sequence having at least 90% identity to the nucleotide sequence of SEQ ID NO: 1, and encoding a protein having polygalacturonase activity; and
3) a polynucleotide encoding a protein consisting of an amino acid sequence having at least 90% identity to an amino acid sequence of SEQ ID NO: 2, and having polygalacturonase activity.

The gene corresponding to the PgaB gene of *Aspergillus niger* is preferably a gene that is derived from another filamentous fungus and encodes the polygalacturonase. Examples thereof include glycoside hydrolase family 28 protein of *Aspergillus aculeatus* [*Aspergillus aculeatus* ATCC 16872] (accession number XM_020201835), putative extracellular endopolygalacturonase of *Aspergillus japonicus* [*Aspergillus japonicus* CBS 114.51] (accession number XM_025671777), CAZyme family GH28 of *Penicillium roqueforti* [*Penicillium roqueforti*] (accession number XM_039076692), and the like.

The PgaI gene of *Aspergillus niger* is registered as accession number XM_025596768 in the NCBI public database. Specific examples of the PgaI gene of *Aspergillus niger* or the gene corresponding to the PgaI gene include the following 4) to 6):
4) a polynucleotide consisting of a nucleotide sequence of SEQ ID NO: 3;
5) a polynucleotide consisting of a nucleotide sequence having at least 90% identity to the nucleotide sequence set forth in SEQ ID NO: 3, and encoding a protein having polygalacturonase activity; and
6) a polynucleotide encoding a protein consisting of an amino acid sequence having at least 90% identity to an amino acid sequence of SEQ ID NO: 4, and having polygalacturonase activity.

The gene corresponding to the PgaI gene of *Aspergillus niger* is preferably a gene that is derived from another filamentous fungus and that encodes the polygalacturonase. Examples thereof include putative endopolygalacturonase II of *Aspergillus aculeatus* [*Aspergillus aculeatinus* CBS 121060] (accession number XM_025643861), putative endopolygalacturonase II of *Aspergillus japonicus* [*Aspergillus japonicus* CBS 114.51] (accession number XM_025668037), and the like.

The PgaII gene of *Aspergillus niger* is registered as accession number XM_025602343.1 in the NCBI public database. Specific examples of the PgaII gene of *Aspergillus niger* or the gene corresponding to the PgaII gene include the following 7) to 9):
7) a polynucleotide consisting of a nucleotide sequence of SEQ ID NO: 5;
8) a polynucleotide consisting of a nucleotide sequence having at least 90% identity to the nucleotide sequence of SEQ ID NO: 5, and encoding a protein having polygalacturonase activity; and
9) a polynucleotide encoding a protein consisting of an amino acid sequence having at least 90% identity to an amino acid sequence of SEQ ID NO: 6, and having polygalacturonase activity.

The gene corresponding to the PgaII gene of *Aspergillus niger* is preferably a gene that is derived from another filamentous fungus and encodes the polygalacturonase. Examples thereof include *Aspergillus aculeatus* ATCC 16872 uncharacterized protein of *Aspergillus aculeatus* (ASPACDRAFT_41690) (accession number XM_020200977.1), *Aspergillus japonicus* CBS 114.51 putative endopolygalacturonase II (BO86DRAFT_431298) (accession number XM_025675761.1), and the like.

"Pectin lyase" is an enzyme which catalyzes the reaction of converting pectin into an oligosaccharide with an unsaturated bond between the C-4 and C-5 terminal residues (EC4.2.2.10). Examples of the pectin lyase gene of the present invention include one or more genes selected from the group consisting of PelD gene and PelF gene.

Such a pectin lyase gene is preferably derived from a filamentous fungus, and examples include those derived from microorganisms of the genus *Aspergillus* (e.g., *Aspergillus niger, Aspergillus japonicus,* and *Aspergillus ludhuensis*), microorganisms of the genus *Penicillium* (e.g., *Penicillium digitatum* and *Penicillium solitum*), microorganisms of the genus *Talaromyces* (e.g., *Talaromyces rugulosus* and *Talaromyces cellulolyticus*), and the like.

Preferred among these is PelD gene of *Aspergillus niger* or a gene corresponding to the PelD gene.

The PelD gene of *Aspergillus niger* is registered as accession number XM_025604039.1 in the NCBI database. Examples of the PelD gene of *Aspergillus niger* or the gene corresponding to the PelD gene include the following 10) to 12):
10) a polynucleotide consisting of a nucleotide sequence of SEQ ID NO: 7;
11) a polynucleotide consisting of a nucleotide sequence having at least 90% identity to the nucleotide sequence of SEQ ID NO: 7, and encoding a protein having pectin lyase activity; and
12) a polynucleotide encoding a protein consisting of an amino acid sequence having at least 90% identity to an amino acid sequence of SEQ ID NO: 8, and having pectin lyase activity.

The gene corresponding to the PelD gene of *Aspergillus niger* is preferably a gene that is derived from another filamentous fungus and encodes the pectin lyase. Examples thereof include *Aspergillus luchuensis* uncharacterized protein of *Aspergillus luchuensis* (AKAW2_50059S) (accession number XM_041689835.1), *Aspergillus japonicus* CBS 114.51 pectin lyase pelD of *Aspergillus japonicus* (BO86DRAFT_402188) (accession number XM_025673839.1), *Penicillium digitatum* strain Pd01 PL1 mRNA, complete cds of *Penicillium digitatum* (accession number JX298853.1), and the like.

The PelF gene of *Aspergillus niger* is registered as accession number: XM_001401024.2 in the NCBI database. Examples of the PelF gene of *Aspergillus niger* or the gene corresponding to the PelF gene include the following 13) to 15):
13) a polynucleotide consisting of a nucleotide sequence of SEQ ID NO: 9;
14) a polynucleotide consisting of a nucleotide sequence having at least 90% identity to the nucleotide sequence of SEQ ID NO: 9, and encoding a protein having pectin lyase activity; and
15) a polynucleotide encoding a protein consisting of an amino acid sequence having at least 90% identity to an amino acid sequence of SEQ ID NO: 10, and having pectin lyase activity.

The gene corresponding to the PelF gene of *Aspergillus niger* is preferably a gene that is derived from another filamentous fungus and encodes the pectin lyase. Examples thereof include *Aspergillus costaricaensis* CBS 115574 pectin lyase A of *Aspergillus costaricaensis* (BO79DRAFT_237612) (accession number XM_025684723.1), *Aspergillus japonicus* CBS 114.51 pectin lyase 1 of *Aspergillus japonicus* (BO86DRAFT_306335) (XM_025667691.1) (accession number XM_025673839.1), *Penicillium griseofulvum* Pectin lyase fold/virulence factor of *Penicillium griseofulvum* (PGRI_010780) (accession number XM_040788791.1), and the like.

The pectinase gene to be introduced may be one or both of the above polygalacturonase gene and pectin lyase gene; it is preferable to introduce both the polygalacturonase gene and pectin lyase gene, in terms of the biomass viscosity reduction effect.

The means for introducing the polygalacturonase gene and/or pectin lyase gene of the present invention into a filamentous fungus include, for example, introducing a vector or DNA fragment containing the above polynucleotide into a host filamentous fungus (parent strain).

Here, the vector containing the polynucleotide of the present invention is an expression vector, and preferably an expression vector which can introduce the polynucleotide into the host filamentous fungus and express the polynucleotide within the host. Moreover, the vector preferably contains the polynucleotide of the present invention and a control region operably linked thereto. The vector may be a vector capable of autonomous growth and replication outside the chromosome, such as plasmids, or may be a vector integrated into the chromosome.

Specific examples of vectors include pBluescript II SK(-) (Stratagene), pUC-based vectors such as pUC18/19 and pUC118/119 (Takara Bio Inc.), pET-based vectors (Takara Bio Inc.), pGEX-based vectors (GE HealthCare), pCold-based vectors (Takara Bio Inc.), pHY300PLK (Takara Bio Inc.), pUB110 (Mckenzie, T.et al., 1986, Plasmid 15(2): 93-103), pBR322 (Takara Bio Inc.), pRS403 (Stratagene), pMW218/219 (Nippon Gene Co., Ltd.), pRI-based vectors such as pRI909/910 (Takara Bio Inc.), pBI-based vectors (Clontech), IN3-based vectors (Inplanta Innovations Inc.), pPTR1/2 (Takara Bio Inc.), pDJB2 (D. J. Ballance et al., Gene, 36, 321-331, 1985), pAB4-1 (van Hartingsveldt W et al., Mol Gen Genet, 206, 71-75, 1987), pLeu4 (M. I. G. Roncero et al., Gene, 84, 335-343, 1989), pPyr225 (C. D. Skory et al., Mol Genet Genomics, 268, 397-406, 2002), pFG1 (Gruber, F.et al., Curr Genet, 18, 447-451, 1990), and the like.

Examples of the DNA fragment containing the polynucleotide of the present invention include PCR-amplified DNA fragments and restriction enzyme-cleaved DNA fragments. Preferably, the DNA fragment can be an expression cassette containing the polynucleotide of the present invention and a control region operably linked thereto.

The control region contained in the above vector or DNA fragment is a sequence for expressing the gene of the present invention in a host into which the vector or DNA fragment has been introduced. Examples thereof include expression control regions such as promoters and terminators, replication origins, and the like. The type of control region can be appropriately selected depending on the type of host cell into which the vector or DNA fragment is to be introduced. If necessary, the vector or DNA fragment may further have a selection marker, such as an antibiotic-resistant gene or an amino acid synthesis-related gene.

The control region contained in the above vector or DNA fragment is preferably a control region operably linked upstream of the polygalacturonase gene and/or pectin lyase gene, and having the ability to constitutively express or highly express downstream genes (so-called strong control region). Examples of the strong control region include cbh1 promoter, cbh2 promoter, egl1 promoter, xyn1 promoter, xyn2 promoter, xyn3 promoter, and the like of *Trichoderma reesei.*

Further examples of the strong control region include, but are not limited to, the control region of the rRNA operon, the control region of a gene encoding ribosome protein, and the like.

The target polynucleotide and control region contained in the above vector or DNA fragment may be introduced into the nucleus of the host, but may be introduced into the host genome. Alternatively, the target polynucleotide contained in the above vector or DNA fragment may be directly introduced into the host genome and operably linked to a highly expressed promoter on the genome. The means for introducing the polynucleotide into the genome include, for example, a homologous recombination method.

To introduce the vector or DNA fragment into the host cell, a general method of transformation, such as an electroporation method, a transformation method, a transfection method, a conjugation method, a protoplast method, a particle gun method, or an Agrobacterium method, can be used.

The recombinant filamentous fungus into which the target vector or DNA fragment has been introduced can be selected using a selection marker. For example, when the selection marker is an antibiotic-resistant gene, the transformed cell into which the target vector or DNA fragment has been introduced can be selected by culturing cells in a medium supplemented with the antibiotic. Further, for example, when the selection marker is an amino acid synthesis-related gene, after the gene is introduced into an amino acid auxotrophic filamentous fungus strain, the filamentous fungus strain into which the target vector or DNA fragment has been introduced can be selected using the presence or absence of amino acid auxotrophy as an indicator. Alternatively, the introduction of the target vector or DNA fragment can also be confirmed by examining the DNA sequence of the recombinant strain by PCR or the like.

With the above procedure, a recombinant filamentous fungus into which the polygalacturonase gene and/or pectin lyase gene of the present invention has/have been introduced can be produced. The recombinant filamentous fungus of the present invention has the ability to produce cellulase and pectinase.

The filamentous fungus used as the parent strain in the present invention is not limited as long as it is a fungus which produces cellulase as a biomass saccharifying enzyme, and is a filamentous fungus of a different genus or species from the polygalacturonase gene or pectin lyase gene to be introduced. Examples thereof include filamentous fungi belonging to the phyla *Eumycota* and *Oomycota.* Specific examples of such filamentous fungi include those of the genera *Trichoderma, Aspergillus, Penicillium, Neurospora, Fusarium, Chrysosporium, Humicola, Emericella, Hypocrea, Acremonium, Chrysosporium, Myceliophthora, Piromyces, Talaromyces, Thermoascus, Thielavia,* and the like. Preferred among these are filamentous fungi of the genus *Trichoderma.*

Examples of the filamentous fungi of the genus *Trichoderma* include *Trichoderma reesei, Trichoderma longibrachiatum, Trichoderma harzianum, Trichoderma koningii, Trichoderma viride,* and the like; preferably *Trichoderma reesei,* and more preferably *Trichoderma reesei* PCD-10 strain (FERM P-8172) and *Trichoderma reesei* PC-3-7 strain (ATCC66589).

The filamentous fungus as the parent strain may be a wild-type strain, a strain artificially bred from the wild-type strain, or a variant strain (variant) or mutant in which a nucleotide sequence in its genome has been substituted, added, deleted, or modified.

Preferred examples of the recombinant filamentous fungus of the present invention include recombinant filamentous fungi obtained by introducing PgaB gene of *Aspergillus niger* or a gene corresponding to the PgaB gene, or PelD gene of *Aspergillus niger* or a gene corresponding to the PelD gene, into *Trichoderma reesei* PC-3-7 strain (ATCC66589) and its variants, and recombinant filamentous fungi obtained by introducing PgaB gene of *Aspergillus niger* or a gene corresponding to the PgaB gene, and the PelD gene of *Aspergillus niger* or a gene corresponding to the PelD gene into *Trichoderma reesei* PC-3-7 strain (ATCC66589) and its variants. Specific examples include the strains disclosed in the Examples described later.

In thus-established recombinant filamentous fungus strain of the present invention, more pectinases are expressed within the fungal cells than the parent strain. Accordingly, when using this strain to saccharify biomass containing cellulose and pectin, an increase in viscosity of slurry caused by pectin can be reduced compared to the case of saccharification using the parent strain. Specifically, the viscosity of slurry obtained by saccharifying cassava residue using the recombinant filamentous fungus strain of the present invention can be reduced by 50% or more, preferably 60% or more, and more preferably 70% or more, compared to the case of saccharification using the parent strain.

The viscosity is measured, for example, by a B-type viscometer at a solid concentration of from 10 to 20 mass% at 50°C.

Therefore, the use of the recombinant filamentous fungus of the present invention makes it possible to prevent an increase in viscosity and to increase the saccharification efficiency in the saccharification of biomass such as cassava containing a large amount of pectin.

### (3. Production of Biomass Saccharifying Enzyme)

The above recombinant filamentous fungus is cultured in the presence of a cellulase inducer, an enzyme containing cellulase and pectinase is produced and accumulated in the culture, and the enzyme is collected from the culture, whereby a biomass saccharifying enzyme can be produced.

The "cellulase inducer" as mentioned herein is not limited as long as it is a substance which induces the production of cellulase by cellulase-producing filamentous fungi. Examples include cellulose; sophorose; and compounds selected from the group consisting of cello-oligosaccharides, such as cellobiose, cellotriose, cellotetraose, cellopentaose, and cellohexaose.

Cellulose includes polymers in which glucose is polymerized through β-1,4-glucosidic bonds and derivatives thereof. The degree of polymerization of glucose is not particularly limited. Examples of the derivatives include carboxymethylated, aldehydated, or esterified derivatives. Further, cellulose may be β-glucoside, which is a glycoside, lignocellulose, which is a complex with lignin and/or hemicellulose, or a complex with pectin or the like. Cellulose may be crystalline cellulose or non-crystalline cellulose.

The cellulase inducer can be added by any method, such as bulk addition (batch method), divided addition (fed-batch method), or continuous addition (feed method). The amount of cellulase inducer added to the medium may be an amount which allows the filamentous fungus of the present invention to induce the production of cellulase and pectinase, and the amount differs depending on the addition method; however, the total amount is preferably 0.1 mass% or more, more preferably 0.5 mass% or more, and more preferably 1 mass% or more, and is preferably 40 mass% or less, more preferably 35 mass% or less, and more preferably 30 mass% or less, based on the medium. The total amount is also preferably from 0.1 to 40 mass%, more preferably from 0.5 to 35 mass%, and more preferably from 1 to 30 mass%.

Of these, the amount of cellulase inducer added by bulk addition is preferably 0.1 mass% or more, more preferably 0.5 mass% or more, and more preferably 1 mass% or more, and is preferably 16 mass% or less, more preferably 14 mass% or less, and more preferably 12 mass% or less. The amount of cellulase inducer is also preferably from 0.1 to 16 mass%, more preferably from 0.5 to 14 mass%, and more preferably from 1 to 12 mass%.

The medium used in the method of the present invention may be a synthetic medium or a natural medium as long as it contains nutrients necessary for the growth of the filamentous fungus of the present invention and the production of cellulase and pectinase, such as carbon sources, nitrogen sources, inorganic salts, and vitamins.

The carbon source may be any carbon source which can be utilized by the recombinant filamentous fungus of the present invention. Specific examples thereof include the cellulase inducers mentioned above, as well as carbohydrates such as glucose and fructose, alcohols such as ethanol and glycerol, organic acids such as acetic acid, and the like. These can be used singly or in combination of two or more. The carbon source is preferably a carbohydrate such as glucose or fructose, and more preferably glucose. The amount of glucose added in that case is preferably 0.1 mass% or more, more preferably 0.5 mass% or more, and more preferably 2.5 mass% or more, and is preferably 15 mass% or less, more preferably 10 mass% or less, and more preferably 5 mass% or less, based on the medium. The amount of glucose is also preferably from 0.1 to 15 mass%, more preferably from 0.5 to 10 mass%, and more preferably from 2.5 to 5 mass%. As for the amounts of cellulase inducer and glucose in the medium, the mass ratio is preferably from 10:1 to 1:1, and more preferably from 4:1 to 2:1.

Examples of the nitrogen source include ammonia, ammonium salts such as ammonium sulfate, nitrogen compounds such as amines, natural nitrogen sources such as peptone and soy hydrolysate, and the like.

Examples of inorganic salts include potassium phosphate, magnesium sulfate, sodium chloride, ferrous sulfate, potassium carbonate, and the like.

Examples of vitamins include biotin, thiamine, and the like. Further, if necessary, substances required for the growth of the recombinant filamentous fungus of the present invention can be added.

Culture is preferably carried out under aerobic conditions, such as shaking culture or aerated agitation culture. The culture temperature is preferably 10°C or more, more preferably 20°C or more, and more preferably 25°C or more, and is preferably 50°C or less, more preferably 42°C or less, and more preferably 35°C or less. The culture temperature is also preferably from 10 to 50°C, more preferably from 20 to 42°C, and more preferably from 25 to 35°C.

The pH during culturing is from 3 to 9, and preferably from 4 to 5. The culture time is from 10 hours to 10 days, and preferably from 2 to 7 days.

After the completion of culturing, the culture is collected, and optionally subjected to fungal cell crushing treatment using ultrasound, pressurization, or the like, followed by solid-liquid separation by filtration, centrifugation, or the like, and then ultrafiltration, salting-out, dialysis, chromatography, and the like are appropriately combined, whereby a saccharifying enzyme containing cellulase and pectinase can be obtained. The degree of separation and purification is not particularly limited. The culture supernatant and its crudely separated and purified product itself can also be used as biomass saccharifying enzymes.

The term "cellulase" is a generic name for enzymes which degrade cellulose, and includes endoglucanases which cleave from inside the molecule of cellulose (EC 3.2.1.4); exoglucanases which cleave from the reducing end or non-reducing end of cellulose to liberate cellobiose (cellobiohydrolase, EC 3.2.1.91), and β-glucosidase (EC 3.2.1.21).

Further, the term "pectinase" is a generic name for enzymes which degrade pectic substances (polysaccharides composed of α-1,4 bonds of D-galacturonic acid), and include polygalacturonase, pectin lyase, and pectinesterase; however, the saccharifying enzymes of the present invention are preferably polygalacturonase and pectin lyase.

### (4. Saccharification of Biomass)

Due to use of the recombinant filamentous fungus of the present invention, monosaccharides can be produced by degrading and saccharifying biomass. Such a method can be performed by using known techniques.

That is, monosaccharides can be produced by saccharifying biomass through using, as a biomass saccharifying agent, a culture obtained by culturing the recombinant filamentous fungus of the present invention described above in the presence of a cellulase inducer, and allowing the culture and the biomass to coexist in an aqueous medium and heating while stirring or shaking.

In the present invention, examples of biomass include cellulose- and pectin-containing substances, such as cassava, cassava residue, corn, corn hull, apple residue, and citrus peel; preferred is cassava residue.

In the saccharification of biomass, the pH and temperature of the reaction solution each may be within the range in which cellulase and pectinase are not inactivated. In general, when the reaction is performed at normal pressure, the temperature is within the range from 5 to 95°C, and the pH is within the range from 1 to 11.

The biomass saccharification process may be carried out batchwise or continuously.

The present invention also includes the following as exemplary embodiments. However, the present invention is not limited to these embodiments.
<1> A method for producing a biomass saccharifying enzyme, comprising culturing a recombinant filamentous fungus into which one or both of a polygalacturonase gene selected from the following gene group (a) and a pectin lyase gene selected from the following gene group (b) are introduced:
   (a) PgaB gene, PgaI gene, and PgaII gene; and
   (b) PelD gene and PelF gene.
<2> The method according to <1>, wherein the polygalacturonase gene is PgaB gene of *Aspergillus niger* or a gene corresponding to the PgaB gene, and the pectin lyase gene is PelD of *Aspergillus niger* or a gene corresponding to the PelD gene.
<3> The method according to <1> or <2>, wherein the biomass is biomass comprising cellulose and pectin.
<4> The method according to <1> or <2>, wherein the biomass is cassava.
<5> The method according to any one of <1> to <4>, wherein the filamentous fungus is a filamentous fungus which produces cellulase.
<6> The method according to any one of <1> to <5>, wherein the filamentous fungus is a *Trichoderma* filamentous fungus.
<7> The method according to <6>, wherein the filamentous fungus is *Trichoderma reesei.*
<8> A method for biomass saccharification, comprising using, as a biomass saccharifying agent, a culture obtained by culturing a recombinant filamentous fungus into which one or both of a polygalacturonase gene selected from the following gene group (a) and a pectin lyase gene selected from the following gene group (b) are introduced:
   (a) PgaB gene, PgaI gene, and PgaII gene; and
   (b) PelD gene and PelF gene.
<9> The method according to <8>, which prevents an increase in viscosity of slurry.
<10> The method according to <8> or <9>, wherein the filamentous fungus is a filamentous fungus which produces cellulase.
<11> The method according to any one of <8> to <10>, wherein the filamentous fungus is a *Trichoderma* filamentous fungus.
<12> The method according to <11>, wherein the filamentous fungus is *Trichoderma reesei.*
<13> The method according to any one of <1> to <12>, wherein the PgaB gene of *Aspergillus niger* or the gene corresponding to the PgaB gene is any of the following 1) to 3) :
   1) a polynucleotide consisting of a nucleotide sequence of SEQ ID NO: 1;
   2) a polynucleotide consisting of a nucleotide sequence having at least 90% identity to the nucleotide sequence of SEQ ID NO: 1, and encoding a protein having polygalacturonase activity; and
   3) a polynucleotide encoding a protein consisting of an amino acid sequence having at least 90% identity to an amino acid sequence of SEQ ID NO: 2, and having polygalacturonase activity.
<14> The method according to any one of <1> to <12>, wherein the PgaI gene of *Aspergillus niger* or the gene corresponding to the PgaI gene is any of the following 4) to 6):
   4) a polynucleotide consisting of a nucleotide sequence of SEQ ID NO: 3;
   5) a polynucleotide consisting of a nucleotide sequence having at least 90% identity to the nucleotide sequence of SEQ ID NO: 3, and encoding a protein having polygalacturonase activity; and
   6) a polynucleotide encoding a protein consisting of an amino acid sequence having at least 90% identity to an amino acid sequence of SEQ ID NO: 4, and having polygalacturonase activity.
<15> The method according to any one of <1> to <12>, wherein the PgaII gene of *Aspergillus niger* or the gene corresponding to the PgaII gene is any of the following 7) to 9):
   7) a polynucleotide consisting of a nucleotide sequence of SEQ ID NO: 5;
   8) a polynucleotide consisting of a nucleotide sequence having at least 90% identity to the nucleotide sequence of SEQ ID NO: 5, and encoding a protein having polygalacturonase activity; and
   9) a polynucleotide encoding a protein consisting of an amino acid sequence having at least 90% identity to an amino acid sequence of SEQ ID NO: 6, and having polygalacturonase activity.
<16> The method according to any one of <1> to <12>, wherein the PelD gene of *Aspergillus niger* or the gene corresponding to the PelD gene is any of the following 10) to 12):
   10) a polynucleotide consisting of a nucleotide sequence of in SEQ ID NO: 7;
   11) a polynucleotide consisting of a nucleotide sequence having at least 90% identity to the nucleotide sequence of SEQ ID NO: 7, and encoding a protein having pectin lyase activity; and
   12) a polynucleotide encoding a protein consisting of an amino acid sequence having at least 90% identity to an amino acid sequence of in SEQ ID NO: 8, and having pectin lyase activity.
<17> The method according to any one of <1> to <12>, wherein the PelF gene of *Aspergillus niger* or the gene corresponding to the PelF gene is any of the following 13) to 15):
   13) a polynucleotide consisting of a nucleotide sequence of SEQ ID NO: 9;
   14) a polynucleotide consisting of a nucleotide sequence having at least 90% identity to the nucleotide sequence of SEQ ID NO: 9, and encoding a protein having pectin lyase activity; and
   15) a polynucleotide encoding a protein consisting of an amino acid sequence having at least 90% identity to an amino acid sequence of SEQ ID NO: 10, and having pectin lyase activity.

### Examples

### <Example 1: Production of biomass saccharifying enzyme>

### (1) Synthesis of cDNA of Aspergillus niger NBRC 105649 strain

*Aspergillus niger* NBRC 105649 strain was purchased from the National Institute of Technology and Evaluation, Biological Resource Center. The NBRC 105649 strain was inoculated into a pectin medium (1% Pectin, from Citrus (Wako Pure Chemical), 0.14% (NH₄)₂SO₄, 0.2% KH₂PO₄, 0.03% CaCl₂·2H₂O, 0.03% MgSO₄·7H₂O, 0.1% Bacto Polypepton, 0.05% Bacto Yeast Extract, 0.1% Tween 80, 0.1% Trace element 2, 50 mM tartrate buffer (pH 4.0); all percentages are w/v%) . The composition of Trace element 2 is as follows: 6 mg H₃BO₃, 26 mg (NH₄)₆Mo₇O₂₄·4H₂O, 100 mg FeCl₃·6H₂O, 40 mg CuSO₄·5H₂O, 8 mg MnCl₂·4H₂O, and 200 mg ZnCl₂ were made up to 100 mL with distilled water. Shaking culture was performed at 28°C for 3 days, and the cultured fungal cells were collected using a Miracloth (Millipore), then frozen in liquid nitrogen, and crushed using a Multi-Beads Shocker (Yasui Kikai Corporation). In this case, a metal cone was used as the crushing medium, and crushing was performed at 1,700 rpm for 30 seconds. RNA was extracted from the crushed fungal cells using the RNeasy Mini Kit (Qiagen) in accordance with the protocol. Thereafter, the RNA solution was subjected to the SuperScript III First-Strand Synthesis System for RT-PCR (Thermo Fisher Scientific), thereby synthesizing the cDNA of the *Aspergillus niger* NBRC 105649 strain.

### (2) Production of expression vectors for PgaI, PgaII, PgaB, PelD, and PelF

Using, as a template, a plasmid obtained by inserting a region from upstream to downstream of *Trichoderma* reesei-derived xyn2 gene (SEQ ID NO: 11) into the HincII restriction enzyme cleavage site of pUC118 (Takara Bio Inc.), PCR was performed using the Fw primer 1 (SEQ ID NO: 12) and Rv primer 1 (SEQ ID NO: 13) shown in Table 1 to amplify a fragment (A). In addition, using the genomic DNA of *Trichoderma reesei* as a template, PCR was performed using the Fw primer 2 (SEQ ID NO: 14) and Rv primer 2 (SEQ ID NO: 15) shown in Table 1 to amplify an amplified fragment (B). The obtained DNA fragments (A) and (B) were treated in accordance with the protocol of the In-Fusion HD Cloning Kit (Takara Bio Inc.) to construct plasmid pUCf-xyn2 in which cbh1 terminator was linked to xyn2 gene. Further, using this pUCf-xyn2 as a template, PCR was performed using the Fw primer 3 (SEQ ID NO: 16) and Rv primer 3 (SEQ ID NO: 17) shown in Table 1 to amplify a fragment (C), and using the genomic DNA of *Trichoderma reesei* as a template, PCR was performed using the Fw primer 4 (SEQ ID NO: 18) and Rv primer 4 (SEQ ID NO: 19) shown in Table 1 to amplify a fragment (D). These fragments were similarly treated by the In-Fusion HD Cloning Kit to obtain plasmid pUCf-xyn2-pyr4. Subsequently, using this pUCf-xyn2-pyr4 plasmid as a template, PCR was performed using the Fw primer 5 (SEQ ID NO: 20) and Rv primer 5 (SEQ ID NO: 21) shown in Table 1 to amplify a fragment (E), and using the genomic DNA of *Trichoderma reesei* as a template, PCR was performed using the Fw primer 6 (SEQ ID NO: 22) and Rv primer 6 (SEQ ID NO: 23) shown in Table 1 to amplify a fragment (F). These fragments were similarly treated by the In-Fusion HD Cloning Kit to obtain plasmid pUCf-xyn2-pyr4rec.

Using pUCf-xyn2-pyr4rec as a template, PCR was performed using the Fw primer 7 (SEQ ID NO: 24) and Rv primer 7 (SEQ ID NO: 25) shown in Table 1 to amplify a fragment (G). In addition, using the cDNA of the *Aspergillus niger* NBRC 105649 strain as a template, PCR was performed using the Fw primer 8 (SEQ ID NO: 26) and Rv primer 8 (SEQ ID NO: 27), Fw primer 9 (SEQ ID NO: 28) and Rv primer 9 (SEQ ID NO: 29), Fw primer 10 (SEQ ID NO: 30) and Rv primer 10 (SEQ ID NO: 31), Fw primer 11 (SEQ ID NO: 32) and Rv primer 11 (SEQ ID NO: 33), and Fw primer 12 (SEQ ID NO: 34) and Rv primer 12 (SEQ ID NO: 35) shown in Table 1 to amplify fragments (H) to (L), respectively. The fragment (G) and each of the fragments (H) to (L) were treated by the In-Fusion HD Cloning Kit, thereby obtaining pUCf-Pxyn2-pgaI-pyr4rec, pUCf-Pxyn2-pgaII-pyr4rec, pUCf-Pxyn2-pgaB-pyr4rec, pUCf-Pxyn2-pelD-pyr4rec, and pUCf-Pxyn2-pelF-pyr4rec, which were expression vectors for PgaI, PgaII, PgaB, PelD, and PelF, respectively.

**[Table 1]**

| Primers for amplifying gene fragments | | |
|---|---|---|
| Primer name | Sequence | SEQ ID NO: |
| Fw primer 1 | GACAACGTAGCCGAGCTCTTCTTTTGTGATGTGTG | 12 |
| Rv primer 1 | TTTCGCCACGGAGCTTTAGCTGACGGTGATGGAAG | 13 |
| Fw primer 2 | AGCTCCGTGGCGAAAGCCTG | 14 |
| Rv primer 2 | CTCGGCTACGTTGTCATCGT | 15 |
| Fw primer 3 | ATATCTGGTGGATCTCTCTTCTTTTGTGATGTGTG | 16 |
| Rv primer 3 | ACAACGAGAAAAGAACTCGGCTACGTTGTCATCGT | 17 |
| Fw primer 4 | TTCTTTTCTCGTTGTCTCAC | 18 |
| Rv primer 4 | AGATCCACCAGATATGTCAG | 19 |
| Fw primer 5 | TTCTTTTCTCGTTGTCTCAC | 20 |
| Rv primer 5 | CTCGGCTACGTTGTCATCGTCT | 21 |
| Fw primer 6 | GACAACGTAGCCGAGCTCTTCTTTTGTGATGTGTG | 22 |
| Rv primer 6 | ACAACGAGAAAAGAAAATGATACCTACTGATACCG | 23 |
| Fw primer 7 | TAAAGCTCCGTGGCGAAAGC | 24 |
| Rv primer 7 | GTTGATGTCTTCTTGCTTCA | 25 |
| Fw primer 8 | CAAGAAGACATCAACATGCACTCTTACCAGCTTCT | 26 |
| Rv primer 8 | CGCCACGGAGCTTTAGCAAGAAGCACCGGAAGGAA | 27 |
| Fw primer 9 | CAAGAAGACATCAACATGCACTCGTTTGCTTCTCT | 28 |
| Rv primer 9 | CGCCACGGAGCTTTAACAAGAGGCCACCGAAGGGA | 29 |
| Fw primer 10 | CAAGAAGACATCAACATGCATTTCCTCCAGAACGC | 30 |
| Rv primer 10 | CGCCACGGAGCTTTAATCGCTGCAGGAAGCGCCCG | 31 |
| Fw primer 11 | CAAGAAGACATCAACATGAAGTACGCTGCTGCTCT | 32 |
| Rv primer 11 | CGCCACGGAGCTTTACAGGTTACCCTGACCAGCGT | 33 |
| Fw primer 12 | CAAGAAGACATCAACATGAAGTACTCTACTATCTT | 34 |
| Rv primer 12 | CGCCACGGAGCTTTACAGGTTGCCCTGACCGGCGT | 35 |

### (3) Production of transformant

The vectors constructed in (1) above were each transformed into an uracil auxotrophic strain of *Trichoderma reesei* E1AB1 strain (Enzyme and Microbial Technology, volume 82, January 2016, pages 89-95). Transformation was performed by the protoplast PEG method. Transformants were selected in a selective medium (2% glucose, 1.1 M sorbitol, 2% agar, 0.2% KH₂PO₄ (pH 5.5), 0.06% CaCl₂·2H₂O, 0.06% CsCl₂, 0.06% MgSO₄·7H₂O, 0.5% (NH₄)₂SO₄, 0.1% Trace element 1; all percentages are w/v%) . The composition of Trace element 1 is as follows: 0.5 g FeSO₄·7H₂O, 0.2 g CoCl₂, 0.16 g MnSO₄·H₂O, and 0.14 g ZnSO₄·7H₂O were made up to 100 mL with distilled water. After the selected transformants were stabilized by succession, strains in which the target genes were stably maintained were further selected by colony PCR.

### (4) Culture of transformant

The spores of the strains selected in (3) above were inoculated to 2×10⁵ cells/mL into an Avicel medium (1% Avicel (Sigma-Aldrich), 0.14% (NH₄)₂SO₄, 0.2% KH₂PO₄, 0.03% CaCl₂·2H₂O, 0.03% MgSO₄·7H₂O, 0.1% Bacto Polypepton, 0.05% Bacto Yeast Extract, 0.1% Tween 80, 0.1% Trace element 2, 50 mM tartrate buffer (pH 4.0); all percentages are w/v%), and cultured with shaking at 28°C for 5 days. The composition of Trace element 2 is as follows: 6 mg H3BO3, 26 mg (NH₄)₆Mo₇O₂₄·4H₂O, 100 mg FeCl₃·6H₂O, 40 mg CuSO₄·5H₂O, 8 mg MnCl₂·4H₂O, and 200 mg ZnCl₂ were made up to 100 mL with distilled water. The obtained cultures were centrifuged, followed by filter filtration to obtain culture supernatants. The obtained culture supernatants were used for the examination as saccharifying enzyme solutions.

### <Example 2: Saccharification of biomass>

(1) The protein concentration of each saccharifying enzyme solution was quantified by the Bradford method. Using the Quick Start Protein Assay (BioRad) based on the Bradford method, the protein concentration of the supernatant was calculated based on a calibration curve using bovine γ-globulin as a standard protein.
(2) Cassava residue was used as biomass. The dried cassava residue was ground into 100-g portions with a speed control of 10 and a grinding time of 30 seconds using an absolute mill (ABS-W, produced by Osaka Chemical Co., Ltd.). Compositional analysis of the cassava residue was performed at Tokai-Techno Co., Ltd. and Japan Food Research Laboratories. The composition analysis results (wt%) were as follows.
   Glucose 63.1%
   Xylose 5.2%
   Arabinose 2.2%
   Galactose 6.5%
   Lignin 7.4%
   Galacturonic acid 7.0%
   Rhamnose 0.7%
(3) Using the saccharifying enzyme solutions in which six pectinases were expressed obtained in Example 1, a saccharification test was carried out using the ground cassava residue as a substrate. 40 g-dry of the ground cassava residue was weighed in a 500-mL baffled flask, and 0.07 mg-protein of α-amylase (produced by Sigma-Aldrich, α-Amylase from *Bacillus licheniformis* Type XII-A) and Milli-Q water were added thereto so that the substrate concentration was 15 wt%. Thereafter, the flask was thoroughly shaken by hand. The baffled flask was sealed with a cotton plug and covered with aluminum foil, followed by autoclave (LSX-700, produced by Tomy Seiko Co., Ltd.) treatment at 90°C for 2 hours (gelatinization). Thereafter, the flask was allowed to stand at room temperature until the temperature reached around 60°C, followed by shaking with a shaker (PRXY-30-R-3F, produced by PRECI) set at 50°C at 210 rpm for 1 hour. Thereafter, 2.0 mg-protein of glucoamylase (produced by Sigma-Aldrich, Amyloglucosidase from *Aspergillus niger*) and 8 mg-protein of pectinase expression enzyme were added to the cassava residue slurry, followed by reaction at 50°C at 200 rpm for 24 hours.
(4) Measurement of slurry viscosity

The slurry viscosity after the saccharifying enzyme treatment of the cassava residue was measured by using a B-type viscometer VISCOMETER TVB-10 (produced by Toki Sangyo Co., Ltd.) at a rotational speed of 60 rpm with a rotational time of 180 seconds at a temperature of 50°C, and the viscosity values obtained were compared between the six pectinase-expressing enzymes and the enzyme which did not express pectinase. As a result, the viscosity was generally reduced in the pectinase-expressing enzymes compared to the enzyme which did not express pectinase, and the enzyme co-expressing different pectinases showed the highest viscosity reduction.

**[Table 2]**

| | No pectinase | Pga I | Pga II | PgaB | PeID | PeIF | PgaB,PeID |
|---|---|---|---|---|---|---|---|
| mPa.s | 1144.0 | 445.5 | 561.0 | 453.0 | 403.5 | 207.0 | 179.5 |

### <Comparative Example: Preparation of pectinase>

### (1) Synthesis of cDNA of Aspergillus niger NBRC 105649 strain

*A. niger* NBRC 105649 strain was purchased from the National Institute of Technology and Evaluation, Biological Resource Center. The NBRC 105649 strain was inoculated into a pectin medium (1% Pectin, from Citrus (Wako Pure Chemical), 0.14% (NH₄)₂SO₄, 0.2% KH₂PO₄, 0.03% Cacl₂·2H₂O, 0.03% MgSO₄·7H₂O, 0.1% Bacto Peptone, 0.05% Bacto Yeast Extract, 0.1% Trace element, 50 mM tartrate buffer (pH 4.0). Shaking culture was performed at 28°C for 3 days, and the cultured fungal cells were collected using a Miracloth (Millipore), then frozen in liquid nitrogen, and crushed using a Multi-Beads Shocker (Yasui Kikai Corporation). In this case, a metal cone was used as the crushing medium, and crushing was performed at 1,700 rpm for 30 seconds. RNA was extracted from the crushed fungal cells using the RNeasy Mini Kit (Qiagen) in accordance with the protocol. Thereafter, the RNA solution was subjected to the SuperScript III First-Strand Synthesis System for RT-PCR (Thermo Fisher Scientific), thereby synthesizing the cDNA of the NBRC 105649 strain.

### (2) Artificial synthesis of Fusarium oxysporum-derived DNA

*F. oxysporum*-derived pgx1 gene was artificially synthesized based on publicly available DNA information.

Pgx1 is an exopolygalacturonase, which is a type of pectinase, its amino acid sequence is set forth in SEQ ID NO: 41, and Pgx1 gene consists of a nucleotide sequence of SEQ ID NO: 40.

### (3) Production of expression vectors for PgxC, RgxC, Pgx1, PgaB, and PelD

Using *Pichia* expression vector pD912 (ATUM) as a template, PCR was performed using the Fw primer 13 (SEQ ID NO: 42) and Rv primer 13 (SEQ ID NO: 43) shown in Table 3 to amplify a fragment (A). Using the cDNA of the NBRC 105649 strain as a template, PCR was performed using the Fw primer 14 (SEQ ID NO: 44) and Rv primer 14 (SEQ ID NO: 45) shown in Table 1 to amplify an amplified fragment (B). The obtained DNA fragments (A) and (B) were treated in accordance with the protocol of the In-Fusion HD Cloning Kit (Takara Bio Inc.), thereby constructing ligated plasmid pD912-PgxC.

Using the cDNA of the NBRC 105649 strain as a template, PCR was performed using the Fw primer 15 (SEQ ID NO: 46) and Rv primer 15 (SEQ ID NO: 47) shown in Table 3 to amplify an amplified fragment (C). The obtained DNA fragments (A) and (C) were treated in accordance with the protocol of the In-Fusion HD Cloning Kit, thereby constructing ligated plasmid pD912-RgxC.

Using the artificially synthesized *F. oxysporum-*derived pgx1 gene as a template, PCR was performed using the Fw primer 16 (SEQ ID NO: 48) and Rv primer 16 (SEQ ID NO: 49) shown in Table 3 to amplify an amplified fragment (D). The obtained DNA fragments (A) and (D) were treated in accordance with the protocol of the In-Fusion HD Cloning Kit, thereby constructing ligated plasmid pD912-Pgx1.

Using the cDNA of the NBRC 105649 strain as a template, PCR was performed using the Fw primer 17 (SEQ ID NO: 50) and Rv primer 18 (SEQ ID NO: 51) shown in Table 3 to amplify an amplified fragment (E). The obtained DNA fragments (A) and (E) were treated in accordance with the protocol of the In-Fusion HD Cloning Kit, thereby constructing ligated plasmid pD912-PgaB.

Using the cDNA of the NBRC 105649 strain as a template, PCR was performed using the Fw primer 18 (SEQ ID NO: 52) and Rv primer 18 (SEQ ID NO: 53) shown in Table 3 to amplify an amplified fragment (D). The obtained DNA fragments (A) and (D) were treated in accordance with the protocol of the In-Fusion HD Cloning Kit, thereby constructing ligated plasmid pD912-PelD.

PgxC is an exopolygalacturonase, which is a type of pectinase, its amino acid sequence is set forth in SEQ ID NO: 37, and PgxC gene consists of a nucleotide sequence set forth in SEQ ID NO: 36.

In addition, RgxC is an exorhamnogalacturonan hydrolase, which is a type of pectinase, its amino acid sequence is set forth in SEQ ID NO: 39, and RgxC gene consists of a nucleotide sequence set forth in SEQ ID NO: 38.

**[Table 3]**

| Primers for amplifying gene fragments | | |
|---|---|---|
| Primer name | Sequence | SEQ ID NO: |
| Fw primer 13 | TAAAGGGGCGGCCGCTCAAGAG | 42 |
| Rv primer 13 | AGCTTCGGCCTCTCTTTTCT | 43 |
| Fw primer 14 | AGAGAGGCCGAAGCTGTTCCACACTCCAGCAGAGC | 44 |
| Rv primer 14 | GCGGCCGCCCCTTTATTAATTAGATAATGGGCTGT | 45 |
| Fw primer 15 | AGAGAGGCCGAAGCTGGTAATGTGGAAAACAACCA | 46 |
| Rv primer 15 | GCGGCCGCCCCTTTATCAACACCCACTCAACCCAT | 47 |
| Fw primer 16 | AGAGAGGCCGAAGCTGTGGTTGGTACTTCTTCCCG | 48 |
| Rv primer 16 | GCGGCCGCCCCTTTATTAGCCGTTTGTGGTGTCCC | 49 |
| Fw primer 17 | AGAGAGGCCGAAGCTGCTCCTCTCGAGAAGCGCTC | 50 |
| Rv primer 17 | GCGGCCGCCCCTTTATTAATCGCTGCAGGAAGCGC | 51 |
| Fw primer 18 | AGAGAGGCCGAAGCTGTCGGTGTCTCCGGCACTCC | 52 |
| Rv primer 18 | GCGGCCGCCCCTTTATTACAGGTTACCCTGACCAG | 53 |

### (4) Production of transformant

Each of the target gene expression vectors produced in (3) was transformed into *Pichia pastoris* PPS-90102 (ATUM) in accordance with the ATUM's protocol.

### (5) Culture of transformant

*P. pastoris* was cultured in the following manner. Each transformant was inoculated into BMD 1% medium (0.2 M potassium phosphate (pH 6.0), 13.4 g/L yeast nitrogen base, 0.4 mg/L biotin, 1.1% glucose) and cultured with shaking at 28°C. After a certain amount of fungal cells increased, methanol was added to induce expression, and cultures obtained after 3 to 5 days were each confirmed for the enzyme expression level by SDS-PAGE. Then, the cultures were each centrifuged and then filtered to obtain culture supernatants. The obtained culture supernatants were concentrated and used as enzyme solutions for each pectinase (PgxC, RgxC, Pgx1, PgaB, and PelD).

### <Comparative Test Example 1>

Cassava residue was weighed in a 100-mL baffled Erlenmeyer flask so that the dry weight was 4 g, and 0.002 mg-protein/g-substrate of α-amylase (produced by Sigma-Aldrich, α-Amylase from *Bacillus licheniformis* Type XII-A) and Milli-Q water were added thereto so that the substrate concentration was 15 wt%. Thereafter, the content of the baffled flask was thoroughly mixed, and the flask was sealed with a cotton plug and covered with aluminum foil, followed by gelatinization treatment at 90°C for 2 hours (Autoclave LSX-700, produced by Tomy Seiko Co., Ltd.). Thereafter, the flask was cooled to 50°C using a shaker (PRXY-30-R-3F, produced by PRECI) set at 50°C. Then, 0.05 mg-protein/g-substrate of glucoamylase (produced by Sigma-Aldrich, Amyloglucosidase from *Aspergillus niger*) was added thereto so that the total amount of a saccharifying enzyme obtained by culturing the E1AB1 strain in accordance with Example 1 was 0.2 mg-protein/g-substrate, followed by reaction at 50°C at 200 rpm for 18 hours. Thereafter, the viscosity was measured using a tuning-fork vibration viscometer (SV-10H, produced by A&D Co., Ltd.). The slurry after saccharification was not in a liquid state, and the viscosity could not be measured.

### <Comparative Test Example 2>

The same examination as in Test Example 1 was performed, except that 0.14 mg-protein/g-substrate of E1AB1 strain-derived saccharifying enzyme and 0.06 mg-protein/g-substrate of PgxC were added to the substrate after gelatinization treatment. The slurry after saccharification was not in a liquid state, and the viscosity could not be measured.

### <Comparative Test Example 3>

The same examination as in Test Example 1 was performed, except that 0.14 mg-protein/g-substrate of E1AB1 strain-derived saccharifying enzyme and 0.06 mg-protein/g-substrate of RgxC were added to the substrate after gelatinization treatment. The slurry after saccharification was not in a liquid state, and the viscosity could not be measured.

### <Comparative Test Example 4>

The same examination as in Test Example 1 was performed, except that 0.14 mg-protein/g-substrate of E1AB1 strain-derived saccharifying enzyme and 0.06 mg-protein/g-substrate of Pgx1 were added to the substrate after gelatinization treatment. The slurry after saccharification was not in a liquid state, and the viscosity could not be measured.

### <Reference Test Example 1>

The same examination as in Test Example 1 was performed, except that 0.18 mg-protein/g-substrate of E1AB1 strain-derived saccharifying enzyme and 0.02 mg-protein/g-substrate of PgaB were added to the substrate after gelatinization treatment. The viscosity of the slurry after saccharification was 84 mPa·s.

### <Reference Test Example 2>

The same examination as in Test Example 1 was performed, except that 0.14 mg-protein/g-substrate of E1AB1 strain-derived saccharifying enzyme and 0.06 mg-protein/g-substrate of PelD were added to the substrate after gelatinization treatment. The viscosity of the slurry after saccharification was 459 mPa·s. From the above, it was considered that PgaB, PelD, and the like are effective for viscosity reduction.

## Claims

1. A method for producing a biomass saccharifying enzyme, comprising culturing a recombinant filamentous fungus into which one or both of a polygalacturonase gene selected from the following gene group (a) and a pectin lyase gene selected from the following gene group (b) are introduced:
(a) PgaB gene, PgaI gene, and PgaII gene; and
(b) PelD gene and PelF gene.

2. The method according to claim 1, wherein the polygalacturonase gene is PgaB gene of *Aspergillus niger* or a gene corresponding to the PgaB gene, and the pectin lyase gene is PelD of *Aspergillus niger* or a gene corresponding to the PelD gene.

3. The method according to claim 1 or 2,
wherein the biomass is biomass comprising cellulose and pectin.

4. The method according to claim 1 or 2,
wherein the biomass is cassava.

5. The method according to any one of claims 1 to 4, wherein the filamentous fungus is a filamentous fungus which produces cellulase.

6. The method according to any one of claims 1 to 5, wherein the filamentous fungus is *Trichoderma reesei.*

7. A method for biomass saccharification, comprising using, as a biomass saccharifying agent, a culture obtained by culturing a recombinant filamentous fungus into which one or both of a polygalacturonase gene selected from the following gene group (a) and a pectin lyase gene selected from the following gene group (b) are introduced:
(a) PgaB gene, PgaI gene, and PgaII gene; and
(b) PelD gene and PelF gene.

8. The method according to claim 7, which prevents an increase in viscosity of slurry.

9. The method according to claim 7 or 8,
wherein the filamentous fungus is a filamentous fungus which produces cellulase.
